# EUROPEAN PATENT APPLICATION

(11) **EP 2 517 709 A1**
(43) Date of publication of application: **31.10.2012**
(21) Application number: 10838611.1
(22) Date of filing: 24.11.2010
(51) Int. Cl.: A61K 31/427, A61P 31/14

(54) **MEDICAL AND PHARMACEUTICAL USE OF 2', 2-BIS-THIAZOLE NON-NUCLEOSIDE COMPOUNDS AS HEPATITIS C VIRUS INHIBITOR**

(30) Priority: 23.12.2009 CN 200910200581
(71) Applicant: Shanghai Institute of Materia Medica, Pudong, Shanghai (CN)
(72) Inventor: NAN, Fajun, Shanghai 201203 (CN); ZUO, Jianping, Shanghai 201203 (CN); ZHANG, Yangming, Shanghai 201203 (CN); JI, Feihong, Shanghai 201203 (CN); CHEN, Haijun, Shanghai 201203 (CN); TONG, Xiankun, Shanghai 201203 (CN)
(74) Representative: HOFFMANN EITLE
(86) International application number: PCT/CN2010/079078
(87) International publication number: WO 2011/076048

(57) **Abstract**

The present invention discloses the pharmaceutical use of a 2',2-bis-thiazole non-nucleoside compound of formula I as an inhibitor of hepatitis C virus (HCV). The 2',2-bis-thiazole non-nucleoside compound can inhibit the replication of HCV, and thus has an anti-HCV activity and is useful for treating hepatitis C.

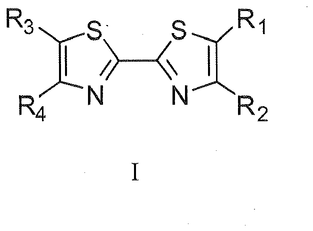

## Description

### Technical Field

The present invention belongs to the filed of pharmacy. In particular, the present invention relates to a class of 2',2-bis-thiazole non-nucleoside compounds having an anti-HCV (Hepatitis C Virus) activity, which can be used in preparing a drug for treating hepatitis C, and thus in treating hepatitis C.

### Background art

Viral hepatitides are internationally recognized tough questions in therapeutics up to now. Also, they are a class of hepatitides that are the most common and harmful with serious infectivity. In general, viruses leading to hepatitis are mainly divided into five types, i.e., types A, B, C, D and E. Among them, hepatitis A and E viruses infect intestinally, and their contamination in water source or food may result in pandemic infection. But hepatitides A and E will neither develop into chronic hepatitis, nor induce hepatocirrhosis, and patients recovering from them will gain a lifelong immunity. Viral hepatitides B, C and D are transmitted through blood, and a few of patients are infected by close contact with patient's saliva, semen, milk or the like, such as the infection between couples or mother and infant. Among the viral hepatitides, more than 80% of the acute infection with hepatitis B virus (HBV), hepatitis C virus (HCV) and hepatitis D virus (HDV) will progress to be chronic, wherein about 20% of them may possibly develop into hepatocirrhosis if suffered from persistent infection, and about 1% to about 5% of them may turn into liver cancer. China has a high morbidity of viral hepatitides, and it is estimated that economic loss directly caused by viral hepatitides is about 30 to 50 billion RMB each year.

Comparing with viral hepatitis B, hepatitis C has a tendency of global prevalence and is the leading cause for the end-stage liver diseases in Europe, America and Japan. According to the statistics of WHO, the global infection rate of HCV is about 3%, and thus it is estimated that about 170 million people have been infected with HCV, and about 35 thousands new cases of hepatitis C develop each year. A nationwide seroepidemiological investigation in China showed that the ratio of a person having anti-HCV antibodies in a general population was 3.2%. Chronic infection of HCV may result in chronic inflammatory necrosis and fibrosis of liver, and a part of the patients may develop into hepatocirrhosis or even hepatocellular carcinoma (HCC). However, there is still short of a desired drug and a therapeutic regimen for hepatitis C to date which seriously threatens the health and life of the patients, and has become a serious social and public health problem. Therefore, China promulgated a guideline for the treatment and prevention of hepatitis C early in 2004.

HBV and HCV belong to different viral genera respectively, although they both belong to the hepatitis virus and have a similar route of infection. HBV belongs to the hepadnaviridae family and is made of a partially double-stranded circular DNA, while HCV belongs to the flaviviridae family and is a positive-sense single-strand RNA virus. At present, clinical anti-HBV drugs are mainly nucleoside drugs, such as lamivudine, famciclovir, lobucavir, adefovir dipivoxiil, FTC (2',3'-dideoxy-5-fluoro-3'-thiacytidine) FMAU (1-(2-fluoro-5-methyl-beta,L-arabinofuranosyl)uracil), FDDC (5-fluoro-2',3'-dideoxycytidine), BMS 200475 (9-[4-hydroxy-3-(hydroxymethyl)-2-methylenecyclopentyl]guanine), enticavir and the like. Since HBV and HCV belong to different viral genera, the existing anti-HBV drugs can not be used for treating Hepatitis C. So far, no effective small molecule drugs for HCV can be used in clinic. Presently, the most effective therapeutic regimen for HCV is the combined administration of interferon and broad-spectrum antiviral agent, such as interferon and ribavirin. But such combined administration only has a sustainable curative effect of less than 40% with serious adverse effect. Therefore, it is of important significance both for clinic and for society to develop novel anti-HCV small molecule drugs.

International application No. PCT/CN2007/001861 (International Publication No. WO 2007/147336), which is a previous international patent application of the present applicant, discloses a class of heterocyclic non-nucleoside compounds, in particularly, discloses the anti-HBV activity of the compounds and their use for treating viral hepatitis B. During the subsequent research, the present inventors further find that among those heterocyclic non-nucleoside compounds, the 2',2-bis-thiazole non-nucleoside compounds also have an anti-HCV activity, and therefore are useful to be developed into anti-HCV drugs. The disclosure of WO 2007/147336 is incorporated herein by its entirety by reference.

### Disclosure of the Invention

Therefore, one object of the present invention is to provide the use of a 2',2-bis-thiazole non-nucleoside compound of formula I in preparing drugs for treating viral hepatitis C.

Another object of the present invention is to provide a method of treating viral hepatitis C.

According to the present invention, a 2',2-bis-thiazole non-nucleoside compound of formula I has an anti-HCV activity, therefore can be used in preparing drugs for treating viral hepatitis C: 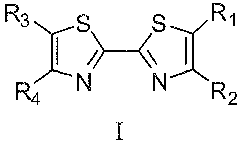 wherein,
R₁ is C₁-C₆ linear or branched alkyl, C₃-C₈ cycloalkyl or benzyl; preferably, R₁ is C₁-C₄ linear or branched alkyl, C₃-C₆ cycloalkyl, or benzyl, such as methyl, ethyl, propyl, butyl, pentyl, hexyl, cyclopropyl, cyclobutyl, cyclopentyl or cyclohexyl; and most preferably, R₁ is isobutyl, n-butyl or benzyl.
R₂ is a group selected from the group consisting of C₁-C₆ hydroxylalkyl; C₁-C₄ alkoxylcarbonyl substituted C₁-C₄ alkyl; C₁-C₄ alkoxylcarbonyl substituted C₂-C₄ alkenyl; and 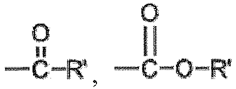 and 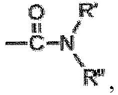 wherein, R' and R" are each independently hydrogen atom, C₁-C₄ linear or branched alkyl, halogenated C₁-C₄ alkyl, phenyl, benzyl, halogenated benzyl, C₁-C₄ alkyl substituted benzyl, C₁-C₄ alkoxyl substituted benzyl, C₁-C₄ alkylamino substituted benzyl, cyano substituted benzyl, carboxyl substituted benzyl, C₁-C₄ alkoxylcarbonyl substituted benzyl, or unsubstituted or C₁-C₄ alkyl substituted 2',2-bis-thiazolylmethylene;
preferably, R₂ is one selected from the group consisting of C₁-C₄ hydroxylalkyl; ethoxylcarbonylethylene; ethoxylcarbonylvinyl; 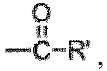 wherein R' is C₁-C₄ linear or branched alkyl; 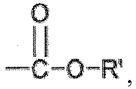 wherein R' is hydrogen atom or C₁-C₄ linear or branched alkyl; and 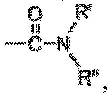 wherein R' and R" are each independently hydrogen atom, C₁-C₄linear or branched alkyl, phenyl, benzyl, fluorobenzyl or 4-[2-(2-thiazolyl)-5-isobutyl-thiazolyl]methylene;
R₃ and R₄ are each independently hydrogen atom, halogen atom, C₁-C₄ linear or branched alkyl, or phenyl; and preferably, R₃ is hydrogen atom, Br, methyl, ethyl or phenyl, and R₄ is hydrogen atom.

The said 2',2-bis-thiazole non-nucleoside compound is further preferably one selected from the group consisting of

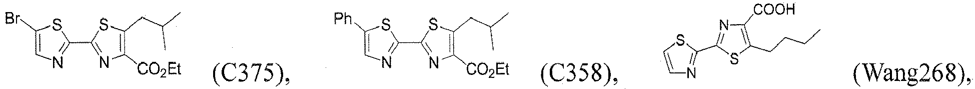

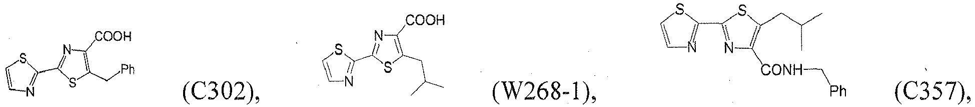

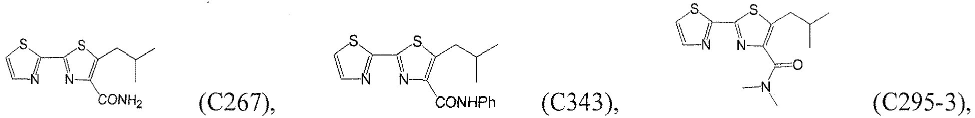

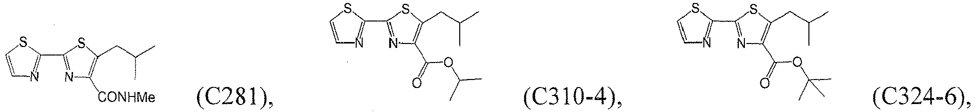

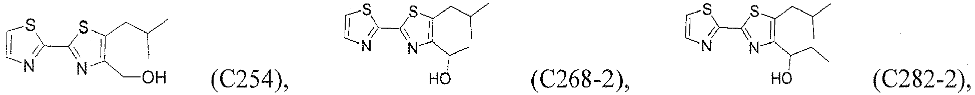

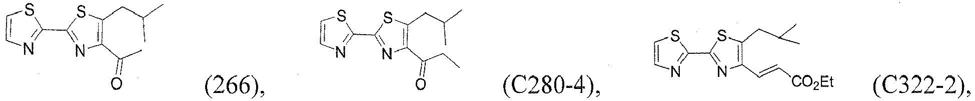

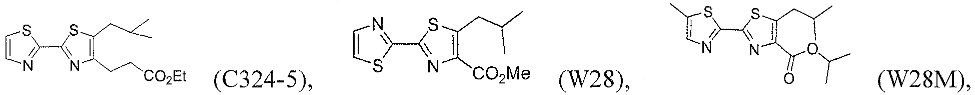

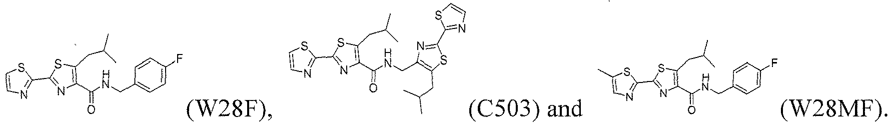

Among them, the most preferable compounds are C322-2, W28MF, C282-2, C503, C267, C280-4, C357, C281, C324-5, C343, C302 and W28F.

The *in vitro* test on anti-HCV activity at cellular level reveals that the 2',2-bis-thiazole non-nucleoside compound of formula I can inhibit the replication of HCV, and thus has the anti-HCV activity, may be useful for treating hepatitis C and used in preparing drugs for treating hepatitis C.

Although the 2',2-bis-thiazole non-nucleoside compound is mainly used for treating HCV, it also can be used for preventing the infection of HCV.

The present invention thus provides a method of treating hepatitis C comprising administering a subject suffering from hepatitis C a therapeutically effective amount of the above 2',2-bis-thiazole non-nucleoside compound.

The 2',2-bis-thiazole non-nucleoside compound may be mixed with pharmaceutically acceptable adjuvants, such as a dispersant, an excipient, a disintegrant, an antioxidant, a sweetening agent, a coating agent, etc., to prepare a pharmaceutical composition. The composition can be prepared by conventional methods in the field of pharmacy, and can be formulated into various routine dosage forms, such as tablet, coating tablet, capsule, powder, etc.

In general, oral administration of the 2',2-bis-thiazole non-nucleoside compound is a preferable route for human. Of course, the drug can be parenterally administered when a patient suffers from deglutition disorder or there is an absorption loss of drug after oral administration.

### Best Mode for Carrying Out the Invention

The *in vitro* anti-HCV activity assay of the 2',2-bis-thiazole non-nucleoside compounds at cellular level was performed by using an anti-HCV activity assay protocol recognized by one skilled in the art.

The anti-HCV activity assay was performed by using Huh7.5.1 cells (presented by WUHAN INSTITUTE of VIROLOGY, CHINESE ACADEMY OF SCIENCES) infected by HCV (J399E) inserted with enhanced green fluorescent protein (EGFP). The variation of the green fluorescence intensity was recorded on a microplate reader and cytotoxicity was measured at the same time, so as to evaluate the influence of the compound on the replication activity of HCV.

Compounds to be tested were dissolved in DMSO to prepare a stock solution before the test. Upon using, the stock solution was diluted to a desired concentration with DMEM (Dulbecco's Modified Eagle Media, purchased from GibcoBRL company (Life Technologies, Grand Island, NY, USA)) containing 10% FBS(fetal bovine serum, purchased from Hyclone company (Logan, Utah, USA)). The final concentration of DMSO for cell culture was less than 0.25 % (V/V, Volume to Volume), and such DMSO concentration would not affect the growth of cells.

Positive control: ribavirin (purchased from Sigma/Aldrich). It was dissolved in DMSO to prepare a stock solution and stored at -20 °C before use, and diluted to a desired concentration upon using.

Huh7.5.1 cells (10% FBS, DMEM) at 10⁵ cells/ml were inoculated on a 96-well plate (Costar 3904) with 100µl per well. 24 h later, the supernatant of the culture media was sucked out, and 50µl of virus supernatant with MOI = 0.1 was added. 8 h later, 50µl of the testing compound solution was added and 100µl of culture media was supplemented. After incubation for 72 h, the supernatant was sucked out, and read on a fluorescent microplate reader (Ex=488nm, Em=516nm). Then each well was added with 20µl of MTT (5mg/ml) and additional 50µl of culture media. 4 h later, 100µl of MTT lysis solution was added. 6 h later, reading was performed at a wavelength of 570nm. Experimental results were shown in Table 1.

**Table 1 Inhibitory activity of some compounds against the replication of HCV**

| Compounds | CC₅₀ (µM) | Replication of HCV | |
|---|---|---|---|
| | | IC₅₀ (µM) | SI |
| C322-2 | >100 | 9.8 | >10.2 |
| W28MF | >100 | 0.8 | >125 |
| C282-2 | >100 | 2.8 | >35.7 |
| C503 | >100 | 1.4 | >71 |
| C267 | >100 | 4.9 | >20.4 |
| C280-4 | >100 | 8.9 | >11.2 |
| C357 | >33.3 | 2.8 | >11.9 |
| C281 | >100 | 13.6 | >7.4 |
| C324-5 | > 100 | 36.6 | >2.7 |
| C343 | >100 | 19.1 | >5.2 |
| C302 | 67.5 | 25.7 | 2.6 |
| W28F | 36.9 | 7.5 | 4.9 |
| ribavirin | 448.8 | 48.3 | 9.3 |

| | | | |
|---|---|---|---|
| Note: CC₅₀ is the concentration of a specimen compound at which 50% of cells was killed, representing the influence of the compound on the growth of Huh7.5.1 cells. IC₅₀ is the concentration of a specimen compound at which 50% of the replication of HCV was inhibited. SI is the bioactivity selective index of a specimen compound, and a SI value of greater than 2 means that the compound is effective, and the larger the SI value is, the more effective the compound will be. | | | |

The above results revealed that under the same experimental conditions, the *in vitro* administration of the above 2',2-bis-thiazole non-nucleoside compounds significantly inhibited the replication of HCV in the infected Huh7.5.1 cells, and the IC₅₀ of the above 2',2-bis-thiazole non-nucleoside compounds ranges from 0.8µM to 36.6µM, which is superior to that of the positive control ribavirin (48.3µM), exhibiting an strong inhibitory activity on replication of HCV *in vitro.* Some of the compounds have excellent SI values, for example, compound W28MF has a SI value of larger than 125.

The 2',2-bis-thiazole non-nucleoside compounds are novel non-nucleoside small molecular compounds, and may have a new anti-HCV mechanism which is different from that of ribavirin and interferon. Meanwhile, the above results of the inhibitory activity assay against the replication of HCV at cellular level indicate that such compounds have a prospect of being applied in the treatment of hepatitis C.

## Claims

1. Use of a 2',2-bis-thiazole non-nucleoside compound of formula I in preparing a drug for treating viral hepatitis C: 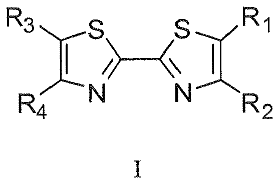 wherein,
R₁ is C₁-C₆ linear or branched alkyl, C₃-C₈ cycloalkyl or benzyl;
R₂ is a group selected from the group consisting of C₁-C₆ hydroxylalkyl, C₁-C₄ alkoxylcarbonyl substituted C₁-C₄ alkyl, C₁-C₄ alkoxylcarbonyl substituted C₂-C₄ alkenyl, 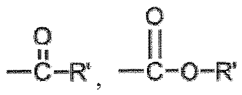 and 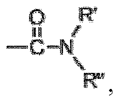 wherein, R' and R" are each independently hydrogen atom, C₁-C₄ linear or branched alkyl, halogenated C₁-C₄ alkyl, phenyl, benzyl, halogenated benzyl, C₁-C₄ alkyl substituted benzyl, C₁-C₄ alkoxyl substituted benzyl, C₁-C₄ alkylamino substituted benzyl, cyano substituted benzyl, carboxyl substituted benzyl, C₁-C₄ alkoxylcarbonyl substituted benzyl, unsubstituted or C₁-C₄ alkyl substituted 2',2-bis-thiazolylmethylene;
R₃ and R₄ are each independently hydrogen atom, halogen atom, C₁-C₄ linear or branched alkyl or phenyl.

2. The use according to claim 1, wherein R₁ is C₁-C₄ linear or branched alkyl, C₃-C₆ cycloalkyl or benzyl.

3. The use according to claim 2, wherein R₁ is isobutyl, n-butyl or benzyl.

4. The use according to claim 1, wherein R₂ is a group selected from the group consisting of C₁-C₄ hydroxylalkyl; ethoxylcarbonylethylene; ethoxylcarbonylvinyl; 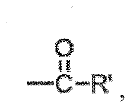 wherein R' is C₁-C₄ linear or branched alkyl; 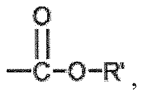 wherein R' is hydrogen atom or C₁-C₄ linear or branched alkyl; and 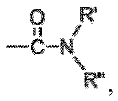 wherein R' and R" are each independently hydrogen atom, C₁-C₄ linear or branched alkyl, phenyl, benzyl, fluorobenzyl or 4-[2-(2-thiazolyl)-5-isobutyl-thiazolyl]methylene.

5. The use according to any one of claims 1 to 4, wherein R₃ is hydrogen atom, Br, methyl, ethyl or phenyl; R₄ is hydrogen atom.

6. The use according to claim 1, wherein the said 2',2-bis-thiazole non-nucleoside compound is selected from the group consisting of

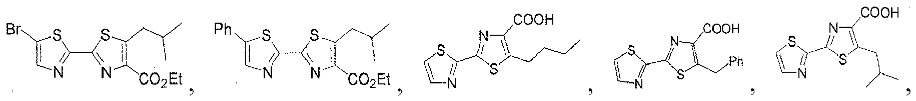

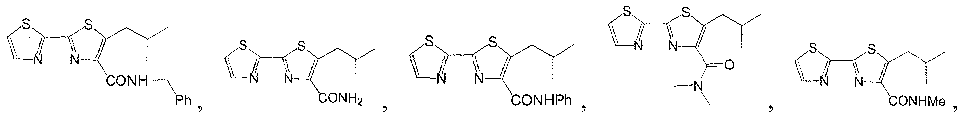

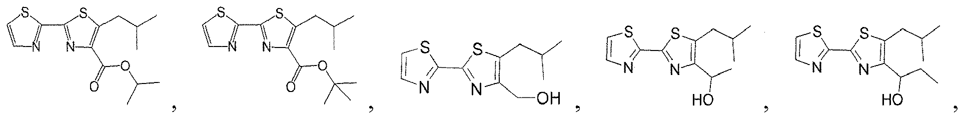

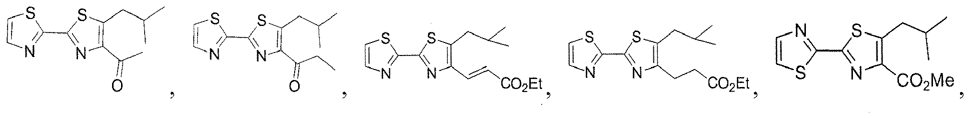

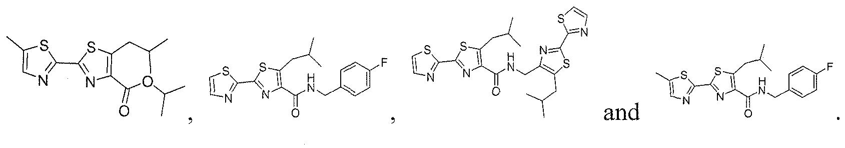

7. The use according to claim 1 or 6, wherein the said 2',2-bis-thiazole non-nucleoside compound is selected from the group consisting of

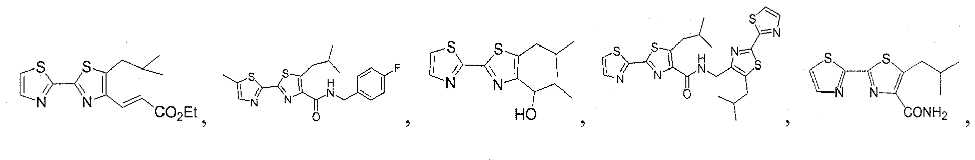

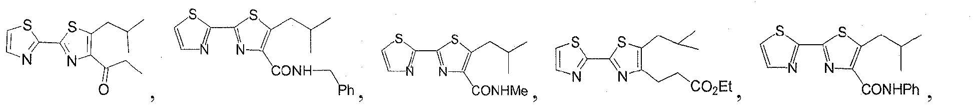

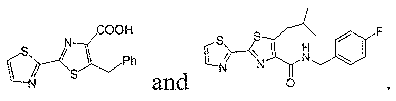

8. A method of treating viral hepatitis C comprising administering to a subject suffering from hepatitis C an therapeutically effective amount of a 2',2-bis-thiazole non-nucleoside compound of formula I: 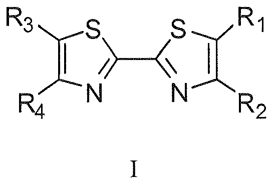 wherein,
R₁ is C₁-C₆ linear or branched alkyl, C₃-C₈ cycloalkyl or benzyl;
R₂ is a group selected from the group consisting of C₁-C₆ hydroxylalkyl, C₁-C₄ alkoxylcarbonyl substituted C₁-C₄ alkyl, C₁-C₄ alkoxylcarbonyl substituted C₂-C₄ alkenyl, 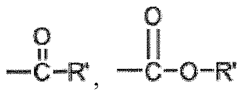 and 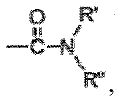 wherein, R' and R" are each independently hydrogen atom, C₁-C₄ linear or branched alkyl, halogenated C₁-C₄ alkyl, phenyl, benzyl, halogenated benzyl, C₁-C₄ alkyl substituted benzyl, C₁-C₄ alkoxyl substituted benzyl, C₁-C₄ alkylamino substituted benzyl, cyano substituted benzyl, carboxyl 1 substituted benzyl, C₁-C₄ alkoxylcarbonyl substituted benzyl, unsubstituted or C₁-C₄ alkyl substituted 2',2-bis-thiazolylmethylene;
R₃ and R₄ are each independently hydrogen atom, halogen atom, C₁-C₄ linear or branched alkyl or phenyl.

9. The method according to claim 8, wherein R₁ is C₁-C₄ linear or branched alkyl, C₃-C₆ cycloalkyl or benzyl;
R₂ is a group selected from the group consisting of C₁-C₄ hydroxylalkyl; ethoxylcarbonylethylene; ethoxylcarbonylvinyl; 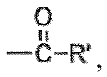 wherein R' is C₁-C₄ linear or branched alkyl; 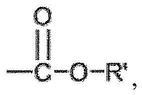 wherein R' is hydrogen atom or C₁-C₄ linear or branched alkyl; and 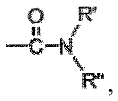 wherein R' and R" are each independently hydrogen atom, C₁-C₄ linear or branched alkyl, phenyl, benzyl, fluorobenzyl or 4-[2-(2-thiazolyl)-5-isobutyl-thiazolyl]methylene;.
R₃ is hydrogen atom, Br, methyl, ethyl or phenyl;
R₄ is hydrogen atom.

10. The method according to claim 8, wherein the said 2',2-bis-thiazole non-nucleoside compound is selected from the group consisting of
